Europäisches Patentamt

(19) European Patent Office

Office européen des brevets

(11) Publication number: **0 132 284**
**B1**

(12) **EUROPEAN PATENT SPECIFICATION**

(45) Date of publication of patent specification: **20.04.88**

(21) Application number: **84303951.2**

(22) Date of filing: **12.06.84**

(51) Int. Cl.⁴: **A 61 F 2/38,** A 61 F 2/46,
A 61 B 17/00

(54) **Apparatus for locating an elbow prothesis.**

(30) Priority: **24.06.83 US 507378**

(43) Date of publication of application:
**30.01.85 Bulletin 85/05**

(45) Publication of the grant of the patent:
**20.04.88 Bulletin 88/16**

(84) Designated Contracting States:
**AT BE CH DE FR GB IT LI SE**

(56) References cited:
**GB-A-1 601 576**
**GB-A-2 022 422**
**GB-A-2 104 392**
**US-A-4 383 337**

(73) Proprietor: **QUEEN'S UNIVERSITY AT
KINGSTON
Kingston Ontario K7L 3N6 (CA)**

(72) Inventor: **Saunders, Gerald A.B.
R.R. No. 1
Sydenham Ontario (CA)**
Inventor: **Sorbie, Charles
208 Alwington Place
Kingston Ontario, K7L 4P8 (CA)**

(74) Representative: **Ben-Nathan, Laurence Albert
et al
Urquhart-Dykes & Lord 91 Wimpole Street
London W1M 8AH (GB)**

Courier Press, Leamington Spa, England.

## Description

This invention relates to an apparatus for locating a prosthesis in an elbow joint.

There are numerous medical conditions which can only be successfully treated by the surgical replacement of an entire elbow joint with a prosthetic device. While the elbow is a relatively simple hinge joint, the surgical replacement thereof is, nevertheless, not without its problems. Firstly, the prosthesis must be extremely accurately located in both the humerus and ulna, and secondly, the joint must be separated to allow the surgeon room to prepare the ends of the ulna and humerus to receive the prosthesis. If the joint is separated and each bone treated independently of the other, accuracy in locating the prosthesis tends to suffer and furthermore it is relatively difficult to separate the joint sufficient to permit access to the bone ends without causing ligament or soft tissue damage.

It is an object of the invention to provide a novel means to cut away the bone, from the medial side, to prepare with extreme accuracy the site in both the ulna and humerus to receive the prosthesis.

GB—A—2104392 discloses a guide device for accurately locating a bone-cutting tool, the device comprising a U-shaped frame dimensioned to receive between the arms thereof the bone to be cut, and means to clampingly engage said frame to said bone at a selected position thereon.

According to the present invention, there is provided such a guide device, sized for clamping around a patient's elbow where it may be used for guiding a tool in the making of the cuts required for an elbow prosthesis, the guide device being provided for this purpose with:

(a) guide means oscillatable about an axis coincident with the axis of said clamping means whereby a cutting tool, rotatable in said guide means, may be oscillated arcuately about said axis at a selected distance therefrom; and

(b) planar guide means releasably engageable with said frame in a plane perpendicular to said axis, including an arcuate slot disposed at a selected distance from said axis to receive a rotatable cutting tool for oscillatory motion therein.

The invention will be described in more detail hereinafter with reference to the drawings in which:

Figure 1 is a side view of a clamp according to the present invention;

Figure 2 is an end view of a humerus guide; and

Figure 3 is an end view of an ulna guide.

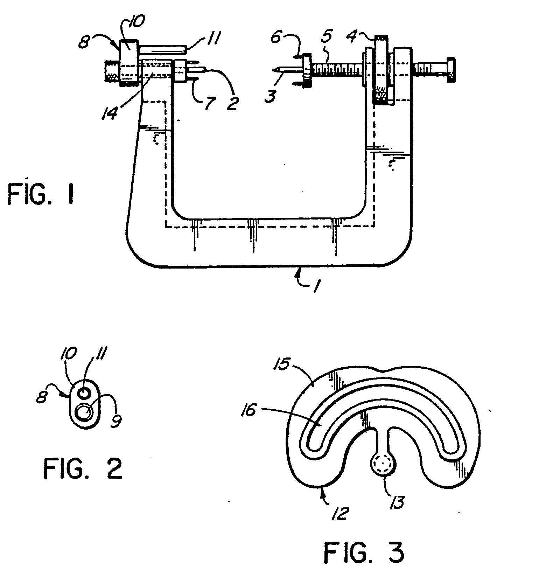

With reference, firstly, to Figure 1, there is shown a U-shaped frame 1, which is used to accurately locate the cutting device on the patient's elbow. Prior to installing the frame on the humerus, a small relatively shallow locating hole is drilled in each side of the humerus at carefully selected positions adjacent but above the lower end thereof using known techniques for location. Pins 2 and 3 are positioned in the locating holes so as to position the frame with pin 3 being on the lateral side of the elbow. The frame is secured in place around the elbow by tightening knurled nut 4, which drives threaded rod 5 forward so that pins 6, 7 are driven into the humerus. Locating pin 2 on the medial side can then be withdrawn and replaced with humerus burr guide 8 (Figure 2). Burr guide 8 comprises a pin 9 mounted perpendicular to the body 10, and a hollow pin 11 axially parallel to and spaced a selected distance from pin 9. Pin 11 is adapted to receive a rotatable burr (not shown) and to oscillate through an arc about the axis of pin 9. The burr may be rotated by a convenient power or air tool as is conventional in an operating theatre. Oscillation of the burr about the axis of pin 9 causes removal of the end of the humerus with an extremely accurately drawn semi-circular cut, so that in end view the end of the humerus presents a convex surface.

Humerus burr guide 8 can now be removed, without distributing the frame 1, and replaced by ulna guide 12 (Figure 3). Ulna guide 12 comprises a pin 13, which is insertable in hole 14 of frame 1, and a plate 15 perpendicular thereto having an arcuate slot 16 therethrough. Slot 16 is spaced a selected radial distance from pin 13 so as to provide the exact spacing required between the humerus and ulna to receive the prosthesis, when the end of the ulna has been removed. A rotary burr (not shown) is inserted through slot 16 and moved arcuately from end to end thereof so as to cut off the end of the ulna and provide a concave surface therein. The frame 1 can now be removed from the patient's elbow, and a further incision made from the lateral side of the elbow so as to expose the end of the radial bone. The entire tip of the radial bone is then removed to provide a flat square end thereto to receive the radial prosthesis.

It will be appreciated that, in certain circumstances, frame 1 can be removed before making the ulna cut and the ulna guide 12 may be attached directly to the elbow solely by means of pin 13.

## Claims

1. A guide device for accurately locating a bone-cutting tool, the device comprising a U-shaped frame (1) dimensioned to receive between the arms thereof the bone to be cut and means (2—7) to clampingly engage said frame to said bone at a selected position thereon; the device being characterised by:

(a) a size which renders it suitable for clamping around a patient's elbow for use in making the cuts required for implantation of an elbow prosthesis;

(b) guide means (8—11) oscillatable about an axis coincident with the axis of said clamping means whereby a cutting tool, rotatable in said guide means, may be oscillated arcuately about said axis at a selected distance therefrom; and

(c) planar guide means (12—15) releasably engageable with said frame in a plane perpendicular to said axis, including an arcuate slot (16) disposed at a selected distance from said axis to receive a rotatable cutting tool for oscillatory motion therein.

2. A guide device as claimed in claim 1 wherein said clamping means includes retractable pin means (2, 3) for insertion into locating holes drilled in the humeral bone of the said elbow.

3. A guide device as claimed in claim 1 or 2 wherein said guide means (8—11) is arranged to receive a humeral bone cutting rotatable burr.

4. A guide device as claimed in claim 1 or 2 wherein said planar guide means (12—15) is arranged to receive an ulna bone cutting rotatable burr.

**Patentansprüche**

1. Führungsgerät zur genauen Positionierung eines Knochenschneidwerkzeuges, wobei das Gerät einen U-förmigen Rahmen (1), der so dimensioniert ist, um zwischen seinen Armen den zu schneidenden Knochen aufzunehmen, und eine Einrichtung (2—7) aufweist, um den Rahmen an dem Knochen in einer ausgewählten Position in klemmendem Eingriff anzubringen, wobei das Gerät gekennzeichnet ist durch:

(a) eine Größe, die es geeignet macht, um es um einen Ellbogen eines Patienten zu klemmen, um es zur Anbringung der für die Implantation einer Ellbogenprothese erforderlichen Schnitte zu verwenden;

(b) eine Führungseinrichtung (8—11), die um eine Achse schwenkbar ist, die mit der Achse der Klemmeinrichtung zusammenfällt, so daß ein Schneidwerkzeug, das in der Führungseinrichtung drehbar ist, bogenförmig um die Achse in einem gewählten Abstand von dieser schwenkbar ist; und

(c) eine ebene Führungseinrichtung (12—15), die mit dem Rahmen in einer Ebene senkrecht zu der Achse in lösbaren Eingriff bringbar ist und die einen bogenförmigen Schlitz (16) aufweist, der in einem gewählten Abstand von der Achse angeordnet ist, um ein drehbares Schneidwerkzeug für die schwenkbare Bewegung in ihr aufzunehmen.

2. Führungsgerät nach Anspruch 1, wobei die Klemmeinrichtung eine zurückziehbare Stiftanordnung (2, 3) zum Einsetzen in Positionierungslöcher aufweist, die in den Humerus des Ellbogens gebohrt sind.

3. Führungsgerät nach Anspruch 1 oder 2, wobei die Führungseinrichtung (8—11) zur Aufnahme eines drehbaren Knochenfräsers zum Schneiden des Humerus angeordnet ist.

4. Führungsgerät nach Anspruch 1 oder 2, wobei die ebene Führungseinrichtung (12—15) zur Aufnahme eines drehbaren Knochenfräsers zum Schneiden der Ulna angeordnet ist.

**Revendications**

1. Un dispositif de guidage pour mise en place précise d'un outil de sectionnement d'os, ce dispositif comprenant un cadre en forme de U (1) dont les dimensions permettent de recevoir entre les bras du U l'os à couper et des moyens (2 à 7) permettant la prise de l'os par serrage dudit cadre sur ledit os dans une position qui est choisie; ce dispositif étant caractérisé par:

(a) une dimension qui le rend adéquat pour enserrer le coude du patient afin de pratiquer les coupes requises pour l'implantation d'une prothèse de coude;

(b) des moyens de guidage (8 à 11) pouvant osciller autour d'un axe qui coïncide avec l'axe desdits moyens de serrage, un outil de coupe tournant dans lesdits moyens de guidage, pouvant osciller en arc autour dudit axe à une distance voulue; et

(c) des moyens de guidage plan (12 à 15) pouvant entrer en prise de façon démontable avec ledit cadre dans un plan perpendiculaire audit axe. et comprenant une fente en arc (16) disposée à une distance donnée dudit axe pour recevoir l'outil de coupe rotatif selon un mouvement oscillant.

2. Un dispositif de guidage selon la revendication 1, dans lequel lesdits moyens de serrage comprennent des moyens de pointes rétractables (2, 3) pour l'insertion dans des trous de localisation percés dans l'humérus dudit coude.

3. Un dispositif de guidage selon l'une des revendications 1 ou 2, dans lequel lesdits moyens de guidage (8 à 11) sont arrangés pour recevoir la fraise tournante de sectionnement de l'humérus.

4. Un dispositif de guidage selon l'une des revendications 1 ou 2, dans lequel lesdits moyens de guidage en plan (12 à 15) sont arrangés pour recevoir la fraise tournante de sectionnement du cubitus.

FIG. 1

FIG. 2

FIG. 3